(19) Europäisches Patentamt
European Patent Office
Office européen des brevets

(11) Publication number: **0 294 021 A1**

# (12) EUROPEAN PATENT APPLICATION

(21) Application number: 88303041.3

(22) Date of filing: 06.04.88

(51) Int. Cl.$^4$: **A61K 35/16**

(30) Priority: 06.04.87 US 34885

(43) Date of publication of application: 07.12.88 Bulletin 88/49

(84) Designated Contracting States: ES GR

(71) Applicant: **BIOGROWTH, INC.**
**Suite 117, 3065 Atlas Road**
**Richmond California 94806(US)**

(72) Inventor: **Spencer, Emerald Martin**
**505 Ortega Street**
**San Francisco California 94112(US)**

(74) Representative: **Paget, Hugh Charles Edward et al**
**MEWBURN ELLIS & CO. 2/3 Cursitor Street**
**London EC4A 1BQ(GB)**

## (54) Human somatomedin carrier protein subunits and process for producing them.

(57) This invention relates to polypeptides that are human somatomedin carrier protein subunits and to processes for producing them. The carrier protein subunits bind to human somatomedin-like polypeptides, also known as insulin-like growth factors. The process involves preparation from a human serum fraction, Cohn IV-1, by a sequence of various chromatographic steps.

EP 0 294 021 A1

# HUMAN SOMATOMEDIN CARRIER PROTEIN SUBUNITS AND PROCESS FOR PRODUCING THEM

## Reference to Related Application

This application is a continuation-in-part of United States patent application serial number 07/034,885, filed April 6 1987.

## STATEMENT AS TO RIGHTS TO INVENTIONS MADE UNDER FEDERALLY-SPONSORED RESEARCH AND DEVELOPMENT

The U.S. Government has a paid-up license in this invention and the right in limited circumstances to require the patent owner to license others on reasonable terms as provided for by the terms of grant nos. SSS-C(3) 1 R43 HD21323-01 ENG awarded by the National Institute of Child Health and Human Development, Department of Health and Human Services.

## Field Of The Invention

This invention relates to human somatomedin carrier protein subunits and to processes for producing them. More particularly, this invention relates to carrier protein subunits that bind to human somatomedin-like polypeptides, also known as insulin-like growth factors. In addition, this invention relates to essentially pure human somatomedin carrier protein subunits. This invention also relates to processes of preparing such carrier protein subunits from human plasma. The process involves preparation from a human serum fraction, Cohn IV-1, by a sequence of various chromatographic steps. The carrier protein subunits and methods of this invention may be used in a variety of therapeutic, diagnostic or other useful applications.

## BACKGROUND OF THE INVENTION

Somatomedins (also sometimes referred to as "SMs") are hormones having useful biological properties. SMs are polypeptides having a molecular weight of approximately 7,500 daltons. SMs (a) mediate the growth-promoting effects of growth hormone (also sometimes referred to as "GH"), (b) have weak insulin-like activity (and for that reason are also called "insulin-like growth factors" or "IGFs"), (c) are mitogenic for a variety of skeletal and other tissues and (d) are transported in plasma bound to a large carrier protein. There are two SM compositions in humans. SM-C is a basic polypeptide and is sometimes referred to as IGF-I. SM-C mediates the growth promoting actions of GH after birth. SM-A is a mixture primarily of a polypeptide known as IGF-II and variable amounts of a modified form of SM-C. Spencer, E.M., et al., "The Identity Of Human Insulin-like Growth Factors I and II With Somatomedins C and A With Rat IGF I and II" in Insulin-like Growth Factors/Somatomedins; ed. Spencer, E.M. (Walter de Gruyter 1983). IGF-II is less GH dependent and may have a role in fetal growth.

SMs may be useful *in vivo* to stimulate bone formation (for example, in treatment of osteoporosis), wound healing, and the growth of animals and GH-deficient humans. Serum levels of SM-C are measured to diagnose acromegaly, pituitary gigantism, GH deficiency, and other growth related conditions. Spencer, E.M., "Somatomedins" in Basic and Clinical Endocrinology, eds. Greenspan F. S. and Forsham, P. H. (1986), p. 89. The Appleton-Century-Crofts SMs are also employed to stimulate *in vitro* the proliferation of a variety of cells in tissue culture and, therefore, are useful in the study of the regulation of normal and abnormal cell growth. SMs produced by certain breast and kidney cancer cells may stimulate the proliferation of both the cancer cells and the vascular and fibrous tissues required to support the growth of the cancer tissues. Spencer, E. M. et al., Possible Auto-stimulation of Human Mammary Carcinoma Growth by Somatomedins. Annals of the N.Y. Acad. Sci., 464, p. 448 (1986): Huff. K.K., et al., Secretion of Insulin-like Growth Factor-I-related Protein by Human Breast Cancer Cells. Cancer Research 46, pp. 4613-4619 (1986). Blocking the action of SMs may be useful to control the growth of these cancers.

Human SMs appear to be transported and regulated *in vivo* by other proteins. Hintz, R. L. et al., "Demonstration of Specific Plasma Protein Binding Sites For Somatomedin," J. Clin. Endocrinol. Metab. 45, p. 988 (1977). These proteins appear to bind to the SMs and regulate the biological activity of the SMs *in*

vivo. Gel filtration of human serum at neutral pH has shown that 95% of the immunoreactive SM-C activity, and probably IGF-II activity, elutes at about 150,000 to 160,000 daltons (150-160 kilodaltons or "kDa") with a minor amount in the range of 35-50 kDa. Only a very small amount of immunoreactive activity elutes at 7.5 kDa, where free SMs should appear. Smith, G. L., Molecular and Cellular Endocrinology 34, p. 83-89 (1984). This indicates that SMs are complexed with larger proteins in plasma.

At least two different classes of proteins or protein complexes in human plasma have been reported to bind SMs. Drop, S. L. et al., "Immunoassay Of A Somatomedin-binding Protein From Human Amniotic Fluid; Levels In Fetal, Neonatal, And Adult Sera", J. Clin. Endocrinol. Metab. 59, p. 908 (1984); Wilkins, J. R. et al., "Affinity-labeled Plasma Somatomedin-C/ Insulin-like Growth Factor I Binding Proteins", J. Clin. Invest. 75, p. 1350 (1985). This description refers to one class of those native proteins or protein complexes as the SM "Carrier Protein" for its function appears to be the transport of SMs. This term is not intended to indicate that the carrier protein is a single protein. There may be more than one carrier protein and it may be a protein complex. This description refers to the other class as the "Amniotic Fluid Binding Protein" or "AFBP". There may be more than one AFBP. It is also possible that additional classes of proteins or protein complexes that bind SMs will be discovered.

Carrier protein activity, like SM-C activity, is GH-dependent, being low in persons with GH deficiency and elevated in patients with GH-producing tumors, a condition known as acromegaly. White, R. M., et al., "The Growth Hormone Dependence Of Somatomedin-binding Protein In Human Serum," J. Clin Endocrinol Metab. 53, p. 49 (1981). The carrier protein displays biological properties indicative of potentially valuable uses. In vivo, when SMs bind to carrier protein, the half-life of the SMs is reported to increase from approximately one hour to up to about 24 hours depending on the animal species tested (Cohen, K. L. et al., "The Serum Half-life Of Somatomedin Activity: Evidence For Growth Hormone Dependence," Acta Endocrinol. 83, p. 243 (1976)), and the SMs are rendered inactive until released. Studies in other model systems suggest that impure preparations containing the carrier protein (a) abolish the metabolic action of the SMs on the perfused rat heart (Meuli C., et al., "NSILA-carrier Protein Abolishes The Action Of Nonsuppressible Insulin-like Activity (NSILA-s) On Perfused Rat Heart", Diabetologia 14, p. 255 (1978)), (b) inhibit the mitogenic effect of the SMs on cells in culture (Knauer, D. J., Proc. Natl. Acad. Sci. U.S.A., 77, pp. 7252-7256 (1980) and Kuffer, A. D., et al., "Partial Purification Of A Specific Inhibitor Of The Insulin-like Growth Factors By Reversed Phase High-performance Liquid Chromatography," J. of Chromatography, 336, pp. 87-92 (1984) and (c) block the insulin-like activity of SMs on rat adipose tissue (Zapf, J., et al., "Inhibition Of The Action Of Nonsuppressible Insulin-like Activity On Isolated Rat Rat Cells By Binding To Its Carrier Protein," J. Clin Invest. 63, p. 1077 (1979). Partially pure preparations of the carrier protein have been used with radiolabeled SMs in research to conduct competitive binding assays for measuring SMs. Moses, A. C., et al., Endocrinology 104, p. 536 (1979).

Because of their valuable biological properties, there have been many efforts to isolate and characterize the carrier protein or the subunits of the carrier protein responsible for that activity. Prior to this invention, all attempts to isolate and characterize in pure form the carrier protein or its active subunits have failed. This is due in part to the low concentration of carrier protein in plasma. A successful purification procedure also had to solve the problems of loss of activity because of enzymatic digestion and instability of the carrier protein, especially to changes in pH. Purification of the carrier protein subunits is further complicated by the presence in plasma of the AFBP, which also binds to somatomedins.

The carrier protein is a glycoprotein. In serum at neutral pH, it is bound with SMs and the complex has a molecular weight of about 150-160 kDa when measured by gel filtration. The molecular weight of the carrier protein complex at neutral pH has also been determined by other methods to be about 125 kDa. Gel filtration chromatography of serum or plasma under acid conditions has been reported to separate bound SMs from the carrier protein and to give rise to a unit of the carrier protein that has a molecular weight of about 40-50 kDa. That unit also binds to somatomedins. Hintz, R. L., et al., "Demonstration Of Specific Plasma Protein Binding Sites For Somatomedin", J. Clin. Endocrinol. Metab. 45, p. 988 (1977). Since the 40-50 kDa acid-stable unit cannot be induced to reform the 150-160 kDa carrier protein complex, others have suggested that the carrier protein may also be composed in part of an acid-labile unit that does not itself bind to somatomedins. Moses, A. C., et al., Endocrinology 104, p. 536 (1979). Furlanetto reported treating serum with a 35-55% ammonium sulfate solution, isolating the precipitate, dissolving the precipitate in 0.05 M Tris, pH 9.20 and chromatographing on DEAE Sephadex A-50 with Tris buffers. Furlanetto, R. W., "The Somatomedin C Binding Protein: Evidence For A Heterologous Subunit Structure", J. Clin, Endocrinol Metab. 51, p. 12 (1980). Furlanetto did not disclose any further purification. Rather, Furlanetto conducted experiments with various fractions to confirm his view that the somatomedin-C binding activity in serum is composed of at least two units, one has a Stokes radius of 36 Å and binds SM-C (the so-called acid stable unit) and the other has Stokes radius of 30-38 Å and does not bind SM-C (the so-called acid labile unit)).

Wilkins identified, by affinity labeling, plasma proteins that complexed with SM-C. Wilkins, J. R., et al., "Affinity-labeled Plasma Somatomedin-C/Insulin-like Growth Factor I Binding Proteins", J. Clin. Invest., 75, p. 1350 (1985). $^{125}$I-SM-C was covalently cross-linked to proteins that bound SM-C in whole plasma and in Sephadex G-200 fractions of plasma. Following sodium dodecylsulfate polyacrylamide gel electrophoresis and autoradiography, the AFBP was identified in addition to species of about 160, 110, 80, 50 and 25 kDa. Wilkins et al. hypothesized that the 160 kDa carrier protein complex consisted of 6 approximately 25 kDa (24-28 kDa) subunit complexes, each composed of the subunit plus SM-C. However, Wilkins et al., did not report isolation or purification of this 25 kDa subunit. Another worker proposed, but did not establish, a slightly larger subunit structure. Daughaday, W. H., et al., "Characterization Of Somatomedin Binding in Human Serum By Ultracentrifugation And Gel Filtration", J. Clin. Endocrinol. Metab. 55, p. 916 (1982).

Several workers have reported unsuccessful attempts to isolate the acid-stable 40-50 kDa carrier protein unit from human plasma. Draznin et al., reported a material containing only 1% SM binding activity and did not disclose whether this material originated from carrier protein or AFBP. Draznin, B., et al., in "Somatomedins and Growth," eds. G. Giordano et al. (Academic Press 1979) pp. 149-160. Fryklund et al., fractionated fresh frozen human plasma by polyethylene glycol precipitation, carboxymethyl-Sephadex chromatography, and gel filtration. Fryklund, L., et al., in Hormones and Cell Culture, eds G. H. Sato et al. (Cold Spring Harbor Laboratory 1979) pp. 49-59. Fryklund et al., proposed that the carrier protein consisted of 2 dissimilar chains of 35 and 45 kDa. Fryklund et al., disclosed that glycine was released by N-terminal sequence analysis, but did not identify from which chain it originated or whether both ended in glycine. The reported binding activity of the Fryklund et al. preparation was very low and purity was not reported. Fryklund et al. did not establish whether the carrier protein or the AFBP was present in their preparation. Morris et al., reported obtaining crude SM binding fractions by acetic acid extraction of human Cohn fraction IV, incubation with $^{125}$I-IGF-I and chromatography on Sephacryl S-200. Morris, D. H., et al., "Structure of Somatomedin-binding Protein: Alkaline pH-Induced Dissociation of an Acid-Stable, 60,000 Molecular Weight Complex Into Smaller Components", Endocrinology 111, pp. 801-805 (1982). Morris et al. described fractions containing bound radio-active IGF-I with apparent molecular weights of 60,000 and 46,000. Morris et al. reported that exposing these fractions to pH 8.0 resulted in a shift of $^{125}$I-IGF-I binding activity from 60,000 and 46,000 daltons to fractions with complexes of 46,000 and 30,000. These fractions were not further purified. Martin et al. reported preparing a polyclonal antibody to the acid-stable unit. The latter was isolated by extracting human Cohn fraction IV with 2 M acetic acid, 75mM NaCl. After removal of SMs by adsorption to SP-Sephadex, the acid stable unit was obtained by IGF-II-Affinity Chromatography and used for immunization. Martin et al. disclosed that HPLC could further purify the acid stable unit. No data was supplied to establish the purity of their final product. Martin, J. L., et al. "Antibody Against Acid-Stable Insulin-Like Growth Factor Binding Protein Detects 150,000 Molecular Weight Hormone-Depedent Complex In Human Plasma", J. Clin. Endocrinol. Metab. 61, pp. 799-801 (1985). Kuffer et al. reported a partial purification of what he described as an inhibitor of insulin-like growth factors (SMs). Kuffer, A. D. et al., "Partial Purification of A Specific Inhibitor of the Insulin-Like Growth Factors By Reverse Phase High-Performance Liquid Chromatography", J. of Chromatography, 336, pp. 87-92 (1984). Kuffer et al. prepared SM inhibitors having a molecular weight of 16,000 to 18,000 from Cohn fraction IV-1 by ion exchange chromatography and sequential gel chromatography under acid conditions on Sephadex G-75 and Bio-Gel P-30 columns. After affinity chromatography and high performance liquid chromatography, Kuffer et al. obtained the "inhibitory activity" as two peaks of activity, corresponding "to a major, apparently homogeneous, protein peak and a minor heterologous peak". Kuffer et al. did not report isolation of the activity of either peak.

None of the above studies disclose a class of human carrier protein subunits capable of binding somatomedin-like polypeptides. In addition, none of these studies disclose any subunits of the carrier protein capable of binding SMs and purified to homogeneity. Purity is required to establish that the carrier protein has been isolated instead of the AFBP or a contaminant and to study biologic activity. An impure preparation may contain enzymes, causing the product to be unstable, and easily degraded or denatured. Impure preparations also cannot be used in animals and humans, because many impurities present in original serum or produced as a result of the purification procedures, are antigenic and could produce unwanted biologic effects. For example, human use in osteoporosis requires removal of all contaminants, which may be antigenic or have adverse biologic effects.

Other workers have isolated a different protein capable of binding SMs and obtained from mid-gestational amniotic fluid of humans, the amniotic fluid binding protein or "AFBP". The AFBP is not the carrier protein or a subunit of the carrier protein. Wilkins, J. R. et al., "Affinity-labeled Plasma Somatomedin-C/Insulin-like Growth factor I Binding Proteins" J. Clin. Invest. 75, p. 1350 (1985). The AFBP (a) is smaller than the so-called acid-stable unit of the carrier protein, having a molecular weight in the range 32-40 kDa,

(b) is not glycosylated, (c) differs from the carrier protein subunits of this invention in its reported N-terminal sequence (Povoa, G. et al., "Isolation And Characterization of A Somatomedin-binding Protein From Mid-term Human Amniotic Fluid", Eur. J. Biochem. 144, pp. 199-204 (1984)), and (d) has different immunologic properties. Drop, S. L. S. et al., "Immunoassay of A Somatomedin-Binding Protein From Human Amniotic Fluid: Levels In Fetal, Neonatal and Adult Sera" J. Clin. Endocrinol. Metab. 59, p. 908 (1984); Martin, J. L. et al., supra, J. Clin. Endocrinol. Metab. 61, pp. 799-801 (1985). Antisera to the AFBP do not cross-react with the 150 kDa carrier protein or its acid-stable unit. Drop et al. reported that the AFBP levels determined by radioimmunoassay (RIA) were found to decrease during infancy and childhood - the inverse of the carrier protein - and also, unlike the carrier protein, to have a significant diurnal variation. Enberg also isolated the AFBP from adult human plasma by four chromatographic steps: CM-Affigel blue, hydroxylapatite, fast protein liquid chromatography gel permeation and high performance liquid chromatography ("HPLC") hydroxyl apatite. Enberg, G., "Purification of A High Molecular Weight Somatomedin Binding Protein From Human Plasma" Biochem. and Biophy. Res. Commun., 135, pp. 178-82 (1986). Enberg reported a "possible" N-terminal sequence, Ala-Pro-Trp-, demonstrating that the AFBP was isolated, not the 150 kDa carrier protein as Enberg erroneously concluded.

Proteins that bind SMs have also been identified in cell culture extracts. Thus far, the carrier protein has not been identified. Spencer first showed that primary cultures of liver cells produced a protein that complexes with SMs. Spencer, E. M, "The Use Of Cultured Rat Hepatocytes To Study The Synthesis Of Somatomedin And Its Binding Protein, " FEBS Letters, 99, p. 157, (1979). Subsequently, several cell types, normal and abnormal, have been found to synthesize a protein that complexes with SMs. Cultured Buffalo rat liver tumor cells (BRL 3A) produce a 33 kDa SM binding protein that differs from the carrier protein by antibody reactivity, N-terminal amino acid sequence, and absence of glycosylation. Lyons R. M. et al., Characterization of Multiplication-Stimulatory Activity "MSA" Carrier Protein", Molecular and Cellular Endocrinol. 45, pp. 263-70 (1986). Mottola. C. et al., J. of Biol. Chem., 261, pp. 1180-88 (1986). Romanus et al. reported that antibodies to this binding protein cross-reacted with a protein present in fetal serum but not adult rat serum. Romanus, J. A. et al., "Insulin-like Growth Factor Carrier Proteins In Neonatal And Adult Rat Serum Are Immunologically Different: Demonstration Using A New Radioimmunoassay For The Carrier Protein From BRL-3A Rat Liver Cells, " Endocrinology, 118, p. 1743 (1986). The BRL-3A binding protein may be the rodent equivalent of the AFBP, but the N-terminal sequence data show no similarity between the two molecules.

## DISCLOSURE OF THE INVENTION

In this description, the following terms are employed:

Somatomedin-like -

A polypeptide displaying the biological activities of one of the human SMs or insulin-like growth factors, including but not limited to SM-C, SM-A, IGF-I and IGF-II. That polypeptide may have amino acids in addition to those of native human SMs or it may not include all the amino acids of native human SMs.

Carrier Protein -

A glycoprotein or complex of glycoproteins in human plasma, displaying the ability to regulate the biological activity of the human SMs in vivo by a process involving binding of the SM-like polypeptides, being growth hormone dependent, and exhibiting an apparent molecular weight of about 125,000-160,000 daltons in physiological pH conditions when complexed with SMs. The carrier protein may also be polymorphic. For example, cells of different individuals may produce carrier protein species which are physiologically similar, but structurally slightly different from the prototype.

Subunit -

A polypeptide fragment, part, or component of a larger protein unit. The term subunit is not confined to its customary meaning of a discrete polypeptide chain bound by covalent or any other types of bonds to another discrete polypeptide chain.

Carrier Protein Subunits -

A class of subunits of the carrier protein.

Polypeptide -

A linear chain of amino acids connected by peptide bonds.

Somatomedin-C ("SM-C" or "IGF-I") -

The principle hormone regulating growth after birth. SM-C mediates the growth promoting action of GH and binds to the carrier protein.

The invention solves the problems referred to by making available human carrier protein subunits capable of binding somatomedin-like polypeptides. The ability of the carrier protein subunits of the invention to bind somatomedin-like polypeptides has been demonstrated by binding those subunits in vitro to somatomedin-C at about physiological pH. This binding activity demonstrates that the carrier protein subunits of the invention will bind somatomedin-like polypeptides in vivo, and provide substantially the transport and regulatory activity of the native carrier protein. When this description refers to the capability of the carrier protein subunits to bind somatomedin-like polypeptides, it is referring to this ability to bind such polypeptides in vitro or in vivo. The carrier protein subunits have no substantial tending activity for insulin.

The carrier protein subunits of the invention each constitute a single polypeptide chain. The carrier protein subunits of the invention have an N-terminal amino acid sequence of the formula:

```
Gly-Ala-Ser-Ser-Ala-Gly-Leu-Gly-Pro-Val-
(1)             (5)                 (10)
Val-Arg-R-Glu-Pro-R-Asp-Ala-Arg-Ala-
                (15)             (20)
Leu-Ala-,
```

wherein R is cysteine or half-cystine. Half-cystine refers to an amino acid bound to another half-cystine amino acid in the same polypeptide chain by a disulfide bond. Because the carrier protein may be polymorphic, the amino acid sequence of the carrier protein subunits may also vary depending on the polymorphic character of the carrier protein. For example, the carrier protein subunits may contain a glycine ("Gly") residue in place of the alanine ("Ala") at position 5 from the N-terminal. Similarly, the Glu at position 14 from the N-terminal may sometimes be replaced in part by Phe.

The carrier protein subunits of the invention have a range of molecular weights. The molecular weights of the carrier protein subunits referred to in this description are those determined by SDS-PAGE gel electrophoresis against proteins of known weight conducted in the presence of a suitable reducing agent such as beta-mercaptoethanol "BME". The known protein standards were 200,000 (myosin (H-chain)), 97,400 (phosphorylase b), 66,200 (bovine serum albumin) 43,000 (ovalbumin), 25,700 (alpha chymotrypsinogen), 18,400 (betalactoglobulin and 14,300 (lysozyme). Carrier protein subunits having molecular weights of about 15,000, 21,000, 26,000 and 30,000 daltons have been isolated and identified. The carrier protein subunits may differ in molecular weight because they were present in the carrier protein as polypeptides of that size or because of enzymatic digestion or break-down from other causes. Whatever the source of these differences, the carrier protein subunits of the invention have a molecular weight of about 30,000 or less. The carrier protein subunits of the invention preferably have a molecular weight of about 15,000 to and including about 30,000 daltons.

The carrier protein subunits of the invention are glycoproteins, as shown by their positive reaction to the

periodic acid Schiff reagent and ability to bind concanavalin A cross-linked to agarose (Con-A Sepharose, Pharmacia). Binding to Con-A Sepharose is specific for glycoproteins containing glucose and mannose residues. Therefore, the carrier protein subunits are substantially glycosylated.

The invention also provides essentially pure carrier protein subunits having SM binding activity. The carrier protein subunits of the invention are essentially free of other proteins, peptides, nucleotides, polysaccharides, lipids and salts. By virtue of the invention, it is possible to obtain those subunits in sufficient purity for use in human and animal therapeutic agents, as animal growth promotion agents, in human and other animal diagnostic reagents, and in human and other animal research applications.

The invention also provides therapeutic compositions comprising an effective amount of at least one carrier protein subunit capable of binding somatomedin-like polypeptides, or pharmacologically acceptable salts thereof, and a pharamacologically acceptable carrier. The carrier protein subunit of such therapeutic compositions may be at least one essentially pure carrier protein subunit. Compositions of carrier protein subunits of the invention have many therapeutic uses involving the important biological properties of SMs. Compositions comprising the human carrier protein subunits may be useful in treatment of diseases involving increased, unregulated SM-dependent growth. Thus, the ability of the carrier protein subunits of the invention to inactivate SMs by binding permits a new therapy of several conditions. In such therapies, it is apparent that an effective amount of the carrier protein subunit is an amount sufficiently in excess of the biologically active, unregulated SMs to block or inactivate the SM activity. For example, an effective amount of carrier protein subunit may be 10 or more times the amount of biologically active SMs on a molar basis. For example, some cancers have been shown to produce SMs: fibrosarcomas, chondrosarcomas and hepatoma cell lines. De Larco, J. E., et al., "A Human Fibrosarcoma Cell Line Producing Multiplication Stimulating Activity "MSA"-related Peptides". Nature, 272, pp. 356-358 (1978). Breast and renal cancers produce a SM which autostimulates the growth of the cancer. Spencer, E. M. et al., "Possible Autostimulation Of Human Mammary Carcinoma Growth By Somatomedins." Annals New York Academy Sciences, 464, pp. 448-449 (1986). Since endothelial cell and fibroblast proliferation are also stimulated by SMs, SMs produced by breast cancers can act also as a paracrine and stimulate the growth of the supporting stromal tissue critical to tumor survival. Bar, R. S., et al., "Receptors For Multiplication-stimulating Activity on Human Arterial and Venous Endothelial Cells, J. Clin. Endocrinol. Metab. 52, p. 814, (1981); Clemmons, D. R., et al., "Hormonal Control Of Immunoreactive Somatomedin Production By Cultured Human Fibroblasts". J. Clin. Invest 67, p. 10 (1981). By blocking the action of SMs, administering the human carrier protein subunit of the invention would be expected to reduce the rate of tumor growth and additionally render the malignant cells more sensitive to other drugs.

Carrier protein subunit therapy could also help prevent blindness secondary to diabetic proliferative retinopathy. Spencer and others have shown that SM-C seems to be one of the factors stimulating endothelial and fibroblast proliferation in diabetic retinopathy. Lorenzi, M., Spencer, E. M. et al., "Improved Diabetic Control, Growth Factors and Rapid Progression Of Retinopathy," New England Journal of Medicine, 308, p. 160, (1983); Ashton, I. K., et al., "Plasma somatomedin in diabetics with retinopathy and joint contractures" in Insulin-Like Growth Factors/ Somatomedins., ed. Spencer, E. M. (Walter de Gruyter). The ability of the carrier protein subunit to block this adverse effect of SM-C (and possibly also IGF-II) could be a useful new therapy.

The carrier protein subunits of the invention are also useful to produce antibodies. The invention will enable pure carrier protein subunits to be used as the antigen to produce both polyclonal antibodies with high titers, high affinities or blocking properties, and monoclonal antibodies that are not now available. These antibodies could be used for immunoassays to make specific measurements, for blocking carrier protein activity, affinity chromatography and immunohistochemistry.

The carrier protein subunits can also be used to develop the first procedure to measure the free level of SMs in body fluids. This method would improve current methods that can only measure total SMs because the free level is really what determines their biological activity. The carrier protein subunit antibody would be used to separate the SM-carrier protein complex from the free SMs in fluids. The free SMs could then be measured by, for example, RIA.

This invention also provides a composition comprising at least one carrier protein subunit substantially complexed with at least one somatomedin-like polypeptide. Such a composition would have a variety of therapeutic applications. SMs possess biological activity which make them potentially useful in many therapeutic applications. However, to maintain the required level of SMs in plasma, multiple daily injections would have to be given because the half-life of SMs may be less than one hour in the free condition. This obstacle cannot be overcome by administering a larger dosage because (a) SMs are potent mitogens for subcutaneous, muscular, and vascular tissues (fibroblasts, endothelial cells, muscle cells, adipocytes, and endothelial cells) and could produce local tissue proliferation, (b) large amounts of free SMs would cause

hypoglycemia, and (c) the excessive amount of SMs required to maintain a steady plasma level would not be cost effective.

SM could be delivered to target tissues in a safe, effective physiologic manner and their half-life significantly prolonged by complexing them to the carrier protein subunits of the invention. The SM in a SM-carrier protein subunit complex would not be mitogenic at injection sites or hypoglycemic. This complex could be formulated to provide controlled, long-term absorption. After transport to target tissues, dissociation would release SM. Thus, therapy would mimic the physiologic delivery system. Successful therapeutic and animal husbandry use of SM-C, IGF-II and other somatomedin-like polypeptides are permitted by a composition of at least one human somatomedin-like polypeptide and at least one carrier protein subunit. Compositions comprising one or more carrier protein subunit and one or more SMs would also be useful for treatment of diseases such as postmenopausal osteoporosis, other forms of osteoporosis, and human GH deficiency, as well as for healing wounds and increasing animal growth. Such composition would be used to deliver SM to bony tissues and stimulate the growth of bone. Dissociation of the SM from the carrier protein subunit-SM complex should stimulate osteoblasts to increase bone formation in postmenopausal osteoporosis, invade the porous matrix of a prosthetic joint thereby stabilizing the prosthesis, and to promote healing of un-united fractures.

Therapeutic compositions comprising an effective amount of at least one carrier protein subunit capable of binding somatomodin-like polypeptides, or pharmacologically acceptable salts thereof, and a pharmacologically acceptable carrier and therapeutic processes using such compositions may also be useful in treating injuries or diseases in which the natural healing mechanism or response involves the presence of regulated levels of biologically active somatomedins. For example, such compositions may be useful in wound healing, where the natural physiological response involves the presence of endogenous SMs at the site of the wound. An effective amount of carrier protein subunit is an amount sufficient to prolong the half-life of the endogenous biologically active somatomedins.

Compositions of at least one carrier protein subunit and SM-C can be used as an effective biodegradable growth-enhancer in animal husbandry. Currently antibiotics and steroids are commercially important animal growth promoters. Because there are serious health concerns with both classes, new agents are being sought, especially biodegradable ones. GH has been investigated. However, the SM-C-carrier protein subunit complex may be much more effective, because SM-C is the direct mediator of the growth promoting effect of GH. SM-C is neither diabetogenic nor lipolytic. For the same reasons applied to postmenopausal osteoporosis, the SM-C would have to be administered in composition with the carrier protein subunit.

For all of these reasons, there have been many attempts to determine the protein structure needed for carrier protein-like activity. None have identified and isolated the carrier protein subunits of this invention or isolated them in pure form.

Another aspect of the invention is a process for producing the human carrier protein subunits from human plasma comprising (a) chromatographing the portions of Cohn fraction IV-1 that are soluble in an aqueous solution of pH of about 4.5 to 7.5 on a sulfopropyl derivative of a cross-linked dextran adsorbent by sequentially eluting with aqueous solutions of increasing pH; (b) chromatographing an acidic solution of pH less than about 4.0 of the fractions from step (a) that contain somatomedin binding activity on the same adsorbent as step (a) and collecting the pass-through fraction, or chromatographing the fractions from step (a) on a phenyl derivative of agarose by adsorption from a neutral solution of about 10% ammonium sulfate and eluting with about 0.5 M sodium thiocyanate solution at about neutral pH; (c) chromatographing the fraction from step (b) containing somatomedin binding activity by gel filtration and eluting with an acidic aqueous solution; (d) chromatographing the fraction from step (c) containing somatomedin binding activity on a solid support cross-linked to substantially pure somatomedin-C by adsorbing at about neutral pH and eluting with an acidic aqueous solution; and (e) chromatographing the fraction from step (d) containing somatomedin binding activity by reverse phase high performance liquid chromatography.

## BEST MODE OF CARRYING OUT THE INVENTION

In order that the invention may be more fully understood, the following detailed description is set forth.

Assay for Somatomedin Binding Activity

The somatomedin binding activity is measured by a protein binding assay employing a radiolabeled $^{125}$I-SM (SM-C or IGF-II) as the ligand. The amount of $^{125}$I-SM bound is compared to that of a standard preparation.

The standard was prepared by gel filtration of a pool of human serum from 10 normal donors. The serum, 35 ml, was added to 35 ml of 4 N acetic acid. After clarification, the sample was chromatographed on Sephadex G-50 (5 × 100 cm) (fractionation range 1,500 to 30,000) equilibrated with 1 M acetic acid at a flow rate of 80 ml/hr. All fractions were assayed for somatomedin binding activity using $^{125}$I SM-C. The binding activity appeared from $K_d$ 0 - 0.4. These fractions were lyophilized, redissolved in 1 M acetic acid and rechromatographed to remove all traces of bound SMs. The final powder was redissolved in 35 ml of 0.1 M phosphate buffer pH 7.0, aliquoted in 100 ul amounts, and stored at -26° C. For each binding assay, a tube of this material was used as a reference that has arbitrarily been assigned a value of 1.0 U/ml.

The assay method was that described by Zapf et al., ("Serum Levels of the Insulin-like Growth Factor (IGF) and its Carrier," Acta Endocrinol. 95, p. 505-517, (1980)). For samples where the carrier protein subunit was still complexed to SMs, the two were separated by Sephadex G-50 chromatography (0.9 × 110 cm) in 1 M acetic acid. The binding activity peak ($K_d$.1 - 0.4) was then lyophilized, reconstituted in assay buffer and tested. For samples that did not contain bound SMs, the samples were either dialyzed against assay buffer and tested directly or, if the concentration of binding activity was low, dialyzed vs 0.1 M acetic acid, lyophilized and dissolved in a smaller volume of assay buffer. The assay buffer was 0.1 M sodium phosphate pH 7.0 containing 0.2% human or bovine serum albumin which had been previously tested to ensure absence of competing activity. SM-C or IGF-II were iodinated by the method of Spencer. Grecu, E. O., E. M. Spencer, et al., "Serum Somatomedin Responser to Human Growth Hormone Infusion in Patients with Diabetes Mellitus; Correlation with the Degree of Control of Diabetes," Am. J. Med. Sci., 287, pp. 7-10 (1984). Serial dilutions (2-or 4-fold) of samples and standard were assayed in triplicate. Assay tubes consisted of 100 ul of $^{125}$I-SM, 20,000 cpm, and 200 ul of the sample. The assay was carried out at 4° C for 16 h although satisfactory results could be obtained with a 2 h incubation at room temperature. The bound $^{125}$I-SM was separated from the free by charcoal extraction. An ice cold solution, 0.8 ml, of 2% activated charcoal with 1% human (or bovine) albumin in 0.1 M phosphate buffer pH 7.0 was added and the tubes vortexed for 15 minutes at 4° C. After centrifugation, the supernatant was counted. The cpm bound were plotted against the log of the dose and the potency of the unknown related to that of the standard assigned a value of 1.0 U/ml. The specificity of binding was determined by incubating the sample with a large excess of an unlabeled SM.

Process For Producing Carrier Protein Subunits

The procedure for producing the carrier protein subunits began with Cohn fraction IV-1. This is a human plasma fraction that contains about 10% of the plasma proteins and 40% of the original plasma carrier protein activity. It is a green-yellow paste, approximately 35% solids, much of which are denatured insoluble proteins and glycoproteins. Each kilogram of this paste contains approximately 10 mg of carrier protein.

All assay buffers described below contained the following enzyme inhibitors, unless otherwise noted: 1 millimolar ("mM") phenylmethylsulfonyl fluoride ("PMSF"), 1 mM N-ethylmaleimide ("NEM"), and 1 mM ethylenediaminetetraacetic acid ("EDTA"). Enzyme inhibitors were essential because either the carrier protein has inherent protease activity or at least one other plasma protease was co-purified through the affinity chromatography step.

Example 1

(a) Ion Exchange Chromatograghy

Fraction IV-1 was handled in 1 kg batches. One kg of fraction IV-1 was added to 10 liters of 40 mM ammonium acetate-acetic acid solution pH 5.65 containing enzyme inhibitors and stirred overnight at 4° C. The suspension was centrifuged and the supernatant was concentrated to about 1 liter by ultrafiltration with a 10,000 MW semipermeable membrane.

The entire concentrate was applied to a 10 × 25 cm column at 4° C of a sulfopropyl derivative of cross-linked dextran (SP-Sephadex, Pharmacia) previously equilibrated with 40 mM ammonium acetate-acetic acid buffer at pH 5.65. The column was washed with 5 liters of the same buffer, followed by 10 liters of 50 mM ammonium acetate pH 6.8, and finally 2 liters of 50 mM ammonium acetate-ammonia at pH 9.6. The pH 9.6 eluate was collected and lyophilized. The recovery of SM binding activity in the lyophilized material determined by the binding assay was 20%. This constituted about a 10 fold purification.

(b) Hydrophobic Interaction Chromatograghy

The lyophilized product with SM binding activity was dissolved in a buffer containing 10% ammonium sulfate and 50 mM tris-(hydroxymethyl) aminomethane ("Tris")-hydrochloride ("Tris-HCl") pH 7.5, dialyzed against the same buffer, and applied to a phenyl agarose column (Phenyl-Sepharose, Pharmacia). The column was eluted first with 1 liter of the same buffer, then with 2 liters of 50 mM Tris-HCl pH 7.5 containing 0.5 M sodium thiocyanate ("NaSCN") and finally with 2 liters of 50 mM Tris. pH 9.0. The eluted fractions were collected and tested for UV absorption at 280 nM and for SM-binding activity in the binding assay. The SM binding activity appeared in the NaSCN fractions. These were lyophilized and then dialyzed against distilled water. A significant amount of precipitate appeared which was separated from the supernatant. This step resulted in a 20-fold purification with 70% recovery.

(c) Gel Filtration

The supernatant was lyophilized, dissolved in 0.5 M acetic acid and chromatographed on a 2 × 100 column of a cross-linked dextran gel (Sephadex G-150, Pharmacia) having a fractionation range of 5,000 - 230,000. Fractions containing SM binding activity were collected. The recovery of SM binding activity was 80-90% by binding assay and the fold purification was 5.

(d) Affinity Chromatography

A SM-C affinity column was first made by coupling SM-C previously purified from human plasma (Spencer et al., in Insulin-Like Growth Factors/Somatomedins, ed. Spencer, E. M., Walter de Gruyter 1983), p. 81) to a hydroxysuccinimidyl derivative of agarose (Affi-Gel 15, BioRad) at pH 8.0 and 25° for 2 hours. The combined carrier protein fractions from the previous step were dialyzed against 0.1 M sodium phosphate pH 7.0, then applied to the SM-C affinity column. After a 15 ml wash with the same buffer, the SM binding activity was eluted with 10 ml of 0.5 M acetic acid and lyophilized.

The SM binding activity was next chromatographed on a cross-linked dextran gel (Sephadex G-100, Pharmacia) having a fractionation range from 4,000 - 90,000 and equilibrated with 0.5 M acetic acid. The fractions containing activity, as shown by the SM binding assay, were lyophilized.

(e) High Performance Liquid Chromatography ("HPLC")

The lyophilized material was chromatographed by HPLC on a butylsilane (Vydac $C_4$ RP (reverse phase)) column. The SM binding activity was eluted by a 0-60% linear gradient of acetonitrile in 0.1% trifluroacetic acid ("TFA"). A sharp peak of SM-C binding activity occurred at 39% acetonitrile and was collected. The SM binding activity in this peak appeared as a single band on 12.5% sodium dodecylsulfate-polyacrylamide gel electrophoresis ("SDS-PAGE") upon staining with a silver stain (BioRad).

The carrier protein subunit isolated had a molecular weight of approximately 26 kDa as shown by SDS-PAGE in the presence of beta-mercaptoethanol. The overall yield of the carrier protein subunit was 4% of the original binding activity.

The N-terminal amino acid sequence of this carrier protein subunit was determined by the method of Hunkapillar and Hood (Methods in Enzymology, 91, p. 486 (1983)), using an automated gas phase sequenator (Beckman 6300) to be:

Gly-Ala-Ser-Ser-Ala-Gly-Leu-Gly-Pro-Val-Val-Arg-R-Glu-Pro-R-Asp-Ala-Arg-Ala-Leu-Ala-,

with R indicating cysteine or half-cystine. This carrier protein subunit bound $^{125}I$ SM-C and was shown to be glycosylated by periodic acid Schiff ("PAS") staining.

Example 2

(a) Ion Exchange Chromatography

One kg of Cohn fraction IV-1 was extracted with 4 liters of 40 mM ammonium acetate-acetic acid buffer pH 5.65 with inhibitors (1 mM EDTA. 1 mM NEM, 0.1 mM PMSF and 1 mg/l aprotinin) overnight at 4° C. The protein solution was spun at 9,000 × g for 30 minutes to separate precipitate from supernatant. The precipitate was reextracted with 4 liters of the above buffer for 4 hours. Supernatants from both extractions were combined.

The supernatants were applied to a SP-Sephadex column (2000 ml resin) which had been equilibrated with the above buffer at 4° C. After application, the column was washed with the same buffer until the $A_{280}$ dropped below 1.0. The column was further washed with 50 mM ammonium acetate buffer, pH 6.8 with inhibitors until the $A_{280}$ was below 1.0. Then the SM binding activity was eluted with 60 mM ammonium acetate-ammonia buffer, pH 9.6 with inhibitors. Finally, the column was cleaned with 60 mM ammonium acetate-ammonia, pH 9.6 with 1.0 M NaCl.

The extract from 1 kg Cohn fraction IV-1 gave about 5,000 units of SM-binding activity. In the pH 9.6 fractions about 7.5% of the activity was recovered, as determined by the binding assay. The weight of the fraction was approximately 5.5 g.

(b) Ion Exchange Chromatography

The pH 9.6 fraction from the previous column was dissolved in 130 ml of a 1 M acetic acid solution containing inhibitors (0.1 mM EDTA, PMSF, NEM and 1 mg/l aprotinin). The solution was dialyzed at 4° C overnight against the same buffer solution and applied to a 5 × 40 cm SP-Sephadex column, which had been previously equilibrated with the same buffer. The column was washed until $A_{280}$ was approximately 0.2, then eluted with 60 mM ammonium acetate-ammonia, pH 9.6, with inhibitors. The SM binding activity was in the pass-through fraction which was dialyzed at 4° C against distilled water overnight to precipitate some denatured proteins. After dialysis, the precipitate was removed by centrifugation at 9,000 × g for 30 minutes and the supernatant freezed-dried. SM binding activity was recovered quantitatively in the soluble pass-through fraction, while SM-C was recovered in the pH 9.6 fraction.

(c) Gel Filtration

An aliquot of the fraction (0.33 g) containing SM binding activity was then dissolved in a minimal amount of 0.5 M acetic acid solution and applied to a 2.5 × 100 cm Sephadex G-100 column, which had been equilibrated under the same conditions. The column was eluted with 0.5 M acetic acid. The $A_{280}$ and SM binding activities of 5 ml fractions were measured. Those fractions exhibiting activity were pooled together and lyophilized. The purification was at this step five fold and the SM binding activity was recovered quantitatively. Several runs were required to process all the material.

(d) Affinity Chromatography

Eighty mg of fractions containing binding activity from the previous step were dissolved in 40 ml of 0.1 M phosphate buffer, pH 7.0, with inhibitors and dialyzed against the same buffer for about 4 hrs. After dialysis, the solution was mixed with 3 ml SM-C-affinity column resin. The mixture was agitated gently at

4°C overnight to increase the binding. The resin was separated from the protein solution by passage through a column. The column was first washed with 50 ml of the phosphate buffer then eluted with 0.5 M acetic acid. The SM binding activity (about 10 units) was dried in a vacuum centrifuge (Speed-Vac Concentrator, Savant Instruments).

(e) HPLC

The 10 units of recovered SM binding activity were dissolved in 1 ml 0.1% TFA solution. After injecting the sample onto a Vydak C4 RP column, the column was eluted with a 0-60% acetonitrile gradient in 60 minutes. The carrier protein peak appeared at approximately 39% acetonitrile, which was collected and lyophilized. The SM binding activity was recovered quantitatively and was approximately 60 micrograms

The SM binding activity appeared after silver staining as a single band on SDS-PAGE, with a molecular weight of about 15 kDa. The overall yield of this example was approximately 3%.

The specific activity of the pure carrier protein subunit was determined to be 4 ug/unit where 1 unit is the amount in 1 ml of a standard plasma prepared from a pool of 10 normal men and women, as described above.

For N-terminal sequence determination, the SM binding activity was denatured and reduced in 4 M guanidine-HCl, 0.5 M Tris-HCl, pH 8.6 and 0.7% $\beta$-mercaptoethanol overnight. Iodoacetamide was added to the solution. The reaction was carried out in the dark for one hour and stopped by adding TFA to 0.1%. The reaction mixture was injected onto the HPLC column and the carboxyamidomethylated carrier protein subunit recovered as before and used for N-terminal sequence analysis. That analysis showed the same N-terminal amino acid sequence described in the example 1.

Example 3.

The carrier protein subunit was purified as in Example 2 through the gel filtration step (c). A 30 mg aliquot of the resulting sample containing SM binding activity was dissolved in 0.1% TFA solution and injected into a preparative Vydak C4 RP column. The column was eluted with a 0-60% acetonitrile gradient in 60 minutes. The SM binding activity peak which eluted at approximately 39% acetonitrile was collected and lyophilized. The SM binding activity was recovered quantitatively.

The sample was subsequently resuspended in a Tris-glycine buffer containing beta-mercaptoethanol and separated by SDS-PAGE (12.5% polyacrylamide). Bands corresponding to 15, 21, 26, and 30 kDa carrier protein subunits (each of which bound labelled SM in a Western blot) were cut from the gel, and the proteins were electroeluted into Tris-glycine buffer. Each of the carrier protein subunits was lyophilized; recoveries were quantitative.

Example 4.

Experiments designed to measure the potential of SM carrier protein subunits to potentiate wound healing were carried out in the following manner. Each of 6 anesthetized 300 gram male Sprague-Dawley rats was implanted subcutaneously (s.c.) with Schilling-Hunt wire mesh wound cylinders in each of the 4 quadrants on their back. Cylindrical chambers, 20 × 1.6 mm i.d. with a volume of 520 ul, were constructed out of stainless steel wire mesh. One end was sealed with wire mesh and the other with a silastic disk. After implantation, the typical progression of wound healing events occurred: thrombosis of blood vessels followed sequentially by migration through the wire mesh of polymorphonuclear leukocytes, macrophages and fibroblasts, with subsequent fibroplasia, collagen synthesis and angiogenesis. During this process, the wound fluid that collected in the hollow chamber could be sampled or injected with active agents (s.c. through the silastic disk). Most of the healing was complete by 17 days after implantation; however, the central cavity was never completely obliterated.

The 15 kDa SM carrier protein subunit was dissolved in PBS (150 mM sodium chloride, 10 mM sodium phosphate, pH 7.4), containing 0.1% bovine albumin. The wound chambers were injected with 100 ul of this solution (containing 1.4 ug of the 15 kDa species) every 12 hours. This amount was selected to be only slightly in excess of the amount of biologically active somatomedins and thereby increase the half-life of somatomedins present. After 17 days, wound cylinders were removed, and the fibrous tissue was scraped carefully from each cylinder. Cylinders injected with 15 kDa carrier protein subunit material were all filled

with dense fibrous tissue that was considerably greater than that in the controls. Specifically, 19.5 ± 7 (SD) mg of protein were deposited in wound chambers containing 15 kDa carrier protein subunit as compared to 7.0 ± 1.6 mg deposited in controls. DNA synthesis was also much greater in carrier protein subunit-containing chambers (1160 ± 200 ug vs 380 ± 15 ug in controls). Likewise, hydroxyproline levels (an indicator of collagen syntehsis) were significantly higher in carrier protein subunit-containing chambers (460 ug vs 270 ug in controls).

These results demonstrate that injection of 15 kDa carrier protein subunit into wound chambers markedly augments the rate of healing.

While we have described certain embodiments of the invention, it is apparent that the basic process and carrier protein subunit can be altered to provide other embodiments which utilize the processes and compositions of the invention. The scope of the invention is defined by the following claims rather than by the specific embodiments that have been presented by way of example.

## Claims

1. A carrier protein subunit capable of binding somatomedin-like polypeptides.

2. A protein subunit of Claim 1 having a molecular weight of about 30,000 daltons or less.

3. A protein subunit of Claim 1 having a molecular weight of about 15,000 to and including about 30,000 daltons.

4. A carrier protein subunit capable of binding somatomedins characterized by an N-terminal amino acid sequence of the formula:

```
Gly-Ala-Ser-Ser-Ala-Gly-Leu-Gly-Pro-Val-
(1)             (5)                 (10)
Val-Arg-R-Glu-Pro-R-Asp-Ala-Arg-Ala-
                (15)            (20)
Leu-Ala-,
```

wherein R is cysteine or half-cystine, the 5-Ala may be Gly, and the 14-Glu may be Phe.

5. A protein subunit of Claim 4 having a molecular weight of about 30,000 daltons or less.

6. A protein subunit of Claim 4 having a molecular weight of about 15,000 to and including about 30,000 daltons.

7. An essentially pure carrier protein subunit capable of binding somatomedin-like polypeptides.

8. An essentially pure carrier protein subunit of claim 7 being essentially free of other proteins, peptides, nucleotides, polysaccharides, lipids and salts.

9. An essentially pure carrier protein subunit of Claim 7 having a molecular weight of about 30,000 daltons or less.

10. An essentially pure carrier protein subunit of Claim 7 having a molecular weight of about 15,000 to and including 30,000 daltons.

11. An essentially pure carrier protein subunit of Claim 7 characterized by an N-terminal amino acid sequence of the formula:

```
Gly-Ala-Ser-Ser-Ala-Gly-Leu-Gly-Pro-Val-
(1)             (5)                 (10)
Val-Arg-R-Glu-Pro-R-Asp-Ala-Arg-Ala-
                (15)            (20)
Leu-Ala-,
```

wherein R is cysteine or half-cystine, the 5-Ala may be Gly and the 14-Glu may be Phe.

12. A therapeutic composition comprising an effective amount of at least one carrier protein subunit according to any one of claims 1 through 11, or a pharmacologically-acceptable salt thereof, and a pharmacologically acceptable carrier.

13. A method for inhibiting the growth of somatomedin-dependent cancers, for inhibiting the effect of somatomedin-C in acromegaly, for inhibiting the growth of retinal blood vessels and fibrous tissues in diabetic retinopathy, for inhibiting growth of tall children, for inhibiting the growth of keloid scars, or for inhibiting the growth of tissue in the orbit of the eyes in malignant exophthalmos, comprising administering an effective amount of a composition according to claim 12.

14. A composition comprising at least one carrier protein subunit according to any one of claims 1 through 11 substantially complexed with at least one human somatomedin-like polypeptide.

15. A method for treating osteoporosis in humans, for stimulating the growth of bone, for stimulating animal growth, for stimulating the healing of human and other animal wounds, for stimulating the growth of patients with growth hormone deficiency, comprising administering an effective amount of a composition of claim 14.

16. Monoclonal and polyclonal antibodies against a carrier protein subunit of claim 1.

17. A method for measuring the level of free somatomedins in human fluids comprising separating somatomedins complexed with the carrier protein subunits of claim 1 from unbound somatomedins.

18. A process for producing a carrier protein subunit from human plasma Cohn fraction IV-1 comprising:

(a) chromatographing the portions of Cohn fraction IV-1 that are soluble in an aqueous solution of pH of about 4.5 to 7.5 on a sulfopropyl derivative of a cross-linked dextran adsorbent by sequentially eluting with aqueous solutions of increasing pH;

(b) chromatographing an acidic solution of pH less than about 4.0 of the fractions from step (a) that contain somatomedin binding activity on the same adsorbent as step (a) and collecting the pass-through fraction or chromatographing the fractions from step (a) on a phenyl derivative of agarose by adsorption from a neutral solution of about 10% ammonium sulfate and eluting with about 0.5 M sodium thiocyanate solution at about neutral pH;

(c) chromatographing the fraction from step (b) containing somatomedin binding activity by gel filtration and eluting with an acidic aqueous solution;

(d) chromatographing the fraction from step (c) containing somatomedin binding activity on a solid support cross-linked to substantially pure somatomedin-c by adsorbing at about neutral pH and eluting with an acidic aqueous solution; and

(e) chromatographing the fraction from step (d) containing somatomedin binding activity by reverse phase high performance liquid chromatography.

19. A method for stimulating the healing of human or other animal wounds comprising administering an effective amount of a composition of claim 12.

European Patent Office

# PARTIAL EUROPEAN SEARCH REPORT

which under Rule 45 of the European Patent Convention shall be considered, for the purposes of subsequent proceedings, as the European search report

Application number
EP 88 30 3041

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | ENDOCRINOLOGY, vol. 111, no. 3, 1982, pages 801-805; The Endocrine Soc., US<br>D.H. MORRIS et al.: "Structure of somatomedin-binding protein: alkaline pH-induced dissociation of an acid-stable, 60,000 molecular weight complex into smaller components"<br>* Page 801, abstract *<br>-- | 1-6 | A 61 K 35/16 |
| Y,D | JOURNAL OF CHROMATOGRAPHY, vol. 336, 1984, pages 87-92; Elsevier Science Publ. B.V. Amsterdam, NL<br>A.D. KUFFER et al.: "Partial purification of a specific inhibitor of the insulin-like growth factors by reversed-phase high-performance liquid chromatography"<br>* Page 87, abstract *<br>--<br>./. | 1-6 | TECHNICAL FIELDS SEARCHED (Int. Cl.4)<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12,14,16-18
Claims searched incompletely:
Claims not searched: 13, 15, 19
Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 07-09-1988 | TURMO BLANCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number
EP 88 30 3041
-2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| Y,D | JOURNAL OF SUPRAMOLECULAR STRUCTURE AND CELLULAR BIOCHEMISTRY, vol. 15, 1981, pages 177-191 D.J. KNAUER et al. "Purification and characterization of multiplication-stimulating activity (MSA) carrier protein"<br>* Page 177, abstract *<br>-- | 1-11 | |
| Y,D | BIOCHEMICAL AND BIOPHYSICAL RESEARCH COMMUNICATIONS, vol. 135, no. 1, February 26, 1986, pages 178-182, Acad. Press, Inc.US G. ENBERG: "Purification of a high molecular weight somatomedin binding protein from human plasma"<br>* Page 178, abstract *<br>-- | 1-11 | TECHNICAL FIELDS SEARCHED (Int. Cl.4) |
| Y | BIOCHEMICAL AND BIOPHYSICAL RESEARCH COMMUNICATIONS, vol. 128, no. 3, May 16, 1985, pages 1071-1078, Acad. Press, Inc. US G. POVOA et al.: "The somatomedin-binding protein isolated from a human hepatoma cell line is identical to the human amniotic fluid somatomedin-binding protein"<br>* Page 1071, abstract; page 1072, "immunoaffinity chromatography" *<br>-- | 1-6,16 | |
| Y,D | JOURNAL OF CLINICAL ENDOCRINOLOGY AND METABOLISM, vol. 59, mp- 5. 1984, The Endocrine. Soc., US S.L.S. DROP, et al.: "Immunoassay of a somatomedin-binding protein from human amniotic fluid: levels in fetal, neonatal, and adult sera"<br>* Page 908, abstract; "Materials and Methods" *<br>-------- | 1-6,16 | |